Europäisches Patentamt

European Patent Office

Office européen des brevets

(19)

(11) **EP 1 458 417 B1**

(12) **EUROPÄISCHE PATENTSCHRIFT**

(45) Veröffentlichungstag und Bekanntmachung des Hinweises auf die Patenterteilung:
**02.11.2005 Patentblatt 2005/44**

(21) Anmeldenummer: **02795020.3**

(22) Anmeldetag: **18.12.2002**

(51) Int Cl.⁷: **A61L 2/00**, A61L 2/08, A61L 2/16, A61L 2/26

(86) Internationale Anmeldenummer:
**PCT/DE2002/004645**

(87) Internationale Veröffentlichungsnummer:
**WO 2003/051404 (26.06.2003 Gazette 2003/26)**

(54) **VERFAHREN ZUR INAKTIVIERUNG VON BAKTERIEN UND LEUKOZYTEN IN THROMBOZYTEN-SUSPENSIONEN**

METHOD FOR INACTIVATING BACTERIA AND LEUKOCYTES IN THROMBOCYTE SUSPENSIONS

PROCEDE D'INACTIVATION DE BACTERIES ET DE LEUCOCYTES DANS DES SUSPENSIONS TROMBOCYTAIRES

(84) Benannte Vertragsstaaten:
**AT BE BG CH CY CZ DE DK EE ES FI FR GB GR IE IT LI LU MC NL PT SE SI SK TR**
Benannte Erstreckungsstaaten:
**AL LT LV MK RO**

(30) Priorität: **19.12.2001 DE 10162712**

(43) Veröffentlichungstag der Anmeldung:
**22.09.2004 Patentblatt 2004/39**

(73) Patentinhaber: **BLUTSPENDEDIENST DER LANDESVERBÄNDE DES DRK NIEDERSACHSEN, SACHSEN-ANHALT, THÜRINGEN, OLDENBURG UND BREMEN GGMBH**
**31830 Springe (DE)**

(72) Erfinder: **MOHR, Harald**
**30177 Hannover (DE)**

(74) Vertreter: **Schupfner, Georg**
**Müller Schupfner**
**Patentanwälte**
**Parkstrasse 1**
**21244 Buchholz (DE)**

(56) Entgegenhaltungen:
**US-A- 5 484 803          US-B1- 6 323 012**

• NYAMEKYE ISAAC ET AL: "Photodynamic therapy of normal and balloon-injured rat carotid arteries using 5-amino-levulinic acid" CIRCULATION, AMERICAN HEART ASSOCIATION, DALLAS, TX, US, Bd. 91, Nr. 2, 1995, Seiten 417-425, XP002202726 ISSN: 0009-7322

• DATABASE BIOSIS [Online] BIOSCIENCES INFORMATION SERVICE, PHILADELPHIA, PA, US; 1994 SVANBERG K ET AL: "Photodynamic therapy on non-melanoma malignant tumours of the skin using topical delta-amino levulinic acid sensitization and laser irradiation." Database accession no. PREV199497374622 XP002236272 & BRITISH JOURNAL OF DERMATOLOGY, Bd. 130, Nr. 6, 1994, Seiten 743-751, ISSN: 0007-0963

• DATABASE BIOSIS [Online] BIOSCIENCES INFORMATION SERVICE, PHILADELPHIA, PA, US; 1993 MALIK Z ET AL: "Collapse of potassium and ionic balance during photodynamic inactivation of leukemic cells, erythrocytes and Staphylococcus aureus." Database accession no. PREV199396139086 XP002236273 & INTERNATIONAL JOURNAL OF BIOCHEMISTRY, Bd. 25, Nr. 10, 1993, Seiten 1399-1406, ISSN: 0020-711X

• DATABASE MEDLINE [Online] US NATIONAL LIBRARY OF MEDICINE (NLM), BETHESDA, MD, US; 1999 TSAI J C ET AL: "Comparative study on the ALA photodynamic effects of human glioma and meningioma cells." Database accession no. NLM10327048 XP002236274 & LASERS IN SURGERY AND MEDICINE. UNITED STATES 1999, Bd. 24, Nr. 4, 1999, Seiten 296-305, ISSN: 0196-8092

• DATABASE BIOSIS [Online] BIOSCIENCES INFORMATION SERVICE, PHILADELPHIA, PA, US; 1992 HANANIA J ET AL: "THE EFFECT OF EDTA AND SERUM ON ENDOGENOUS PORPHYRIN ACCUMULATION AND PHOTODYNAMIC SENSITIZATION OF HUMAN K562 LEUKEMIC CELLS" Database accession no. PREV199294133428 XP002236275 & CANCER LETTERS, Bd. 65, Nr. 2, 1992, Seiten 127-131, ISSN: 0304-3835

**Beschreibung**

Gegenstand der vorliegenden Erfindung ist ein Verfahren zur Inaktivierung von Bakterien und/oder Leukozyten in Thrombozyten-Suspensionen durch den Zusatz von Vorläufer (Precursor)-Verbindungen, aus welchen sich durch endogene Synthese Photosensitizer bilden, und eine photodynamische Behandlung.

Die Dekontamination zellulärer Blutprodukte mittels photodynamischer Verfahren ist bekannt. Eine photodynamische Virusinaktivierung wird bewirkt, indem das zu dekontaminierende Präparat in Lösung bzw. Suspension in Gegenwart einer photoaktiven Substanz, eines Photosensitizers, belichtet wird. Breitere Anwendung findet derzeit nur das photodynamische Verfahren gemäß dem Europäischen Patent 0 491 757-B1 (H. Mohr und B. Lambrecht, Verfahren zur Inaktivierung von Viren in Blut und Blutprodukten). Es wird zur Virusinaktivierung bei Frischplasma eingesetzt. Als photoaktive Substanz dient in der technischen Anwendung vor allem der Phenothiazin-Farbstoff Methylenblau. Es ist weiterhin bekannt, dass Leukozyten in Blutprodukten mittels UV-Bestrahlung abgetötet werden können. Bei Thrombozyten-Suspensionen hat sich hierfür die Bestrahlung mit UV-B (Wellenlängenbereich 290-320 nm) als sinnvoll erwiesen.

Aus Blutspenden werden u.a. folgende für therapeutische Anwendungen wichtige Blutprodukte hergestellt: Frischplasma (FP), Erythrozytenkonzentrate (EK) und Thrombozytenkonzentrate (TK). FP und EK sind in der Regel Einzelspenderpräparate, TK bestehen meist aus den gepoolten Thrombozyten von 4 bis 6 einzelnen Blutspenden. TK sind Gegenstand der vorliegenden Erfindung.

Um die oben genannten Blutprodukte herzustellen, werden die Blutspenden zunächst hochtourig zentrifugiert (mit ca. 2000 bis 5000 g, vorzugsweise etwa 4000g, g = Erdbeschleunigung); dabei trennen sich die Blutbestandteile auf. Die spezifisch schwereren Erythrozyten sammeln sich unten an. Darüber befindet sich eine schmale Schicht, der sogenannte "Buffy Coat", in der die weißen Blutzellen (Granulozyten, Monozyten und Lymphozyten) und die Thrombozyten angereichert sind. Darüber wiederum befindet sich als separate Schicht das Plasma. Die drei Komponenten (EK, FP und der Buffy Coat) werden anschließend in separate Kunststoffbeutel überführt. Zur Herstellung von TK werden 4 bis 6 Buffy Coats gepoolt, in Plasma oder einem speziellen Lagerungsmedium suspendiert und niedrigtourig zentrifugiert. Dabei pelletieren die weißen Blutzellen und restliche Erythrozyten, während sich im Überstand die Thrombozyten finden, entweder in Plasma oder in Lagerungsmedium suspendiert; im letzteren Fall liegt die Plasmakonzentration in der Regel zwischen etwa 30 und 40 %. Dieser Gehalt an Plasma wird derzeit als erforderlich angesehen, damit die TK gelagert werden können.

Eine andere Methode, um aus Blutspenden TK zu gewinnen, besteht darin, die Blutspende niedrigtourig zu zentrifugieren (bis 1000 g, z.B. mit ca. 300 g), so dass die Thrombozyten im überstehenden Plasma verbleiben. Durch einen weiteren Zentrifugationsschritt kann man sie pelettieren und anschließend in einem geringeren Volumen an Plasma oder in Lagerungsmedium mit einem gewissen Plasmagehalt resuspendieren.

TK lassen sich auch durch maschinelle Thrombapherese von Spendern gewinnen, denen hierbei nur die Thrombozyten und eine geringe Menge an Plasma entnommen werden, in dem die Thrombozyten suspendiert sind.

Im Allgemeinen wird kein Wert auf weiße Blutzellen (Leukozyten) in den Blutprodukten gelegt; sie gelten sogar als unerwünscht, weil sie Viren oder Bakterien enthalten und auch selbst bei den Empfängern von Blutprodukten Nebenwirkungen auslösen können.

Beispielsweise können Leukozyten die Ausbildung von Antikörpern gegen fremde HLA-Antigene bewirken. Durch diese sogenannte Allo-Immunisierung werden die Empfänger refraktär gegen Transfusionen weiterer TK von Fremdspendern.

Ein anderes Problem stellen Graft versus Host (GvH)-Reaktionen dar, die durch cytotoxische T-Lymphozyten ausgelöst werden.

Man versucht, die genannten Probleme dadurch zu beseitigen, dass man die Leukozyten durch Filtration entfernt. Bei TK sind die hierfür verwendeten Depletionsfilter aber nicht effektiv genug.

Die Bestrahlung von TK mit UV-B-Licht (Wellenlängenbereich: ca. 290-320 nm) inaktiviert zwar Leukozyten derart, dass die Risiken von GvH-Reaktionen und der Allo-Immunisierung verringert werden, vollständig beseitigen lassen sich diese Risiken aber nicht. Dies gelingt erst durch eine Behandlung mit Gamma- oder Röntgenstrahlung. Hierfür ist der technische und der Sicherheitsaufwand aber so groß, dass die routinemäßige Behandlung aller Präparate nicht in Frage kommt.

Ein weiteres Problem stellt namentlich bei TK eine potenzielle Kontamination mit Bakterien dar, die meist als Folge der Blutspende selbst, aber auch ihrer Prozessierung nach der Spende in die Produkte geraten können. Im Allgemeinen ist zu Beginn das Ausmaß der Verkeimung gering. In FP und EK, die tiefgefroren bzw. bei 4 bis 8 °C gelagert werden, können sich die Bakterien während der Lagerung auch nicht in einem kritischen Ausmaß vermehren. TK müssen hingegen bei ca. 20 °C gelagert werden, damit die Viabilität und die Funktionsfähigkeit der Thrombozyten erhalten bleiben. Die derzeit erlaubte Lagerzeit von TK beträgt bis zu 5 Tage, und in dieser Zeit können sich Bakterien sehr stark vermehren und bei der Transfusion infizierter Präparate ernsthafte septische Reaktionen auslösen. Untersuchungen zufolge sind ca. 0,1 % aller Einzelspender - bzw. ca. 0,7 % aller Pool- TK mit Bakterien kontaminiert. Dabei handelt es sich meist um Gram-positive Hautkeime wie Staphylococcus (S.) epidermidis, S. aureus und Bacillus cereus; es wurden aber auch andere Keime in TK identifiziert, z.B. Streptokokken und Klebsiella (letztere sind Gram-negativ).

Bei der Transfusion von TK kommt es Schätzungen zufolge in einem von 320.000 bis 700.000 Fällen zu einer Sepsis mit tödlichem Ausgang. Derzeit wird bei Blutprodukten (FP, EK und insbesondere TK) das bakterielle Risiko höher eingeschätzt als das virale.

Die US 6,323,012 B1 offenbart z. B. ein Verfahren zur Zerstörung von virusinfizierten Zellen in Blut oder zellulären Bestandteilen in der Gegenwart von 5-Aminolävulinsäure und einer anschließenden Bestrahlung mit Licht der Wellenlängebereiche zwischen 590 und 700 nm. In der US 5,484,803 wird ein ähnliches Verfahren beschrieben, bei dem in der Gegenwart von photosensitiven Substanzen Zielzellen im Blutkreislauf von Tieren transkutan mit Licht im Bereich von 600 und 900 nm bestrahlt werden.

Die Aufgabe der vorliegenden Erfindung bestand darin, eine Methode zu finden, mit der simultan Leukozyten, insbesondere T-Lymphozyten, sowie Bakterien in TK inaktiviert werden können, ohne dass die Funktionen von Thrombozyten und anderen Blutbestandteilen und deren Lagerfähigkeit beeinträchtigt werden. Zudem sollte - was die Wirksamkeit der Methode betrifft - bei den TK die Plasmakonzentration keine Rolle spielen.

Überraschenderweise wurde nun gefunden, dass man in Leukozyten und in Bakterien, offenbar aber nicht in Thrombozyten die Synthese von photoaktiven Substanzen induzieren kann. Die hierdurch zugänglichen photoaktiven Substanzen werden endogene Photosensitizer genannt. Das erfindungsgemäße Verfahren ist durch Anspruch 1 definiert. Vorteilhafte Ausgestaltungen des Verfahrens sind Gegenstand der Unteransprüche.

Die erfindungsgemäß eingesetzten Thrombozyten-Suspensionen sind aufgereinigte, aufkonzentrierte und aus Blut, ggf. auch nur mittelbar, gewonnene Blutprodukte und sind aus Blut oder Blutprodukten erhältlich wie vorstehend für TK beschrieben. Die Thrombozyten der Thrombozyten-Suspensionen können z.B. in Plasma oder in einem Thrombozyten-Lagerungsmedium mit beliebigem PlasmaGehalt suspendiert sein. Als TK werden Thrombozyten-Suspensionen, welche eine Konzentration von mehr als $5x10^8$ Thrombozyten pro ml, besonders bevorzugt von mehr als $10^9$/ml, aufweisen, bezeichnet.

Werden die mit Precursor-Verbindungen vorbehandelten Präparate mit Licht geeigneter Wellenlänge bestrahlt, führt dies zur Abtötung/Deaktivierung von Leukozyten und von Bakterien. Der Grund hierfür wird darin gesehen, dass die Photosensitizer durch das eingestrahlte Licht aktiviert werden und nun ihrerseits gelöste Sauerstoffmoleküle aktivieren. Es entstehen u.a. Singulett -Sauerstoff, Sauerstoffradikale, Hydroxid-Anionenradikale usw., die alle cytotoxisch sind. Ort der Synthese der endogenen Photosensitizer in tierischen Zellen sind die Mitochondrien, in Bakterien das Cytosol. Überraschend bleibt die cytotoxische Wirkung auf die Bereiche beschränkt, in denen der Photosensitizer synthetisiert wurde und sich angereichert hat.

Die Precursor-Verbindungen sind nicht selbst in der oben beschriebenen Weise photoaktiv, sondern fungieren als Startverbindungen, welche erst der weiteren Transformation, z.B. enzymatischen Transformation bedürfen, damit photoaktive Substanzen synthetisiert werden.

Bei den Photosensitizern, deren endogene Synthese induzierbar ist, handelt es sich vorzugsweise um Porphyrinverbindungen, wie Protoporphyrin IX (PPIX) und Coproporphyrin (CP).

Die Synthese von PPIX läuft in den Mitochondrien tierischer Zellen und in einer Reihe von Bakterien nach folgendem bekannten Schema ab:

$$\delta\text{-Aminolävulinsäure} \rightarrow \text{Porphobilinogen} \rightarrow \text{Hydroxymethylbilan} \rightarrow$$

$$\text{Uroporphyrinogen III} \rightarrow \text{Coproporphyrinogen III} \rightarrow$$

$$\text{Protoporphyrinogen IX} \rightarrow \text{PPIX}$$

Thrombozyten verfügen wie tierische Zellen über Mitochondrien. Deshalb war zu erwarten, dass auch in ihnen die Synthese von photoaktiven Porphyrinen induziert wird, wenn man sie in der Gegenwart von Delta-Ala inkubiert. Damit würden sie auch wie tierische Zellen photosensibilisiert und somit inaktiviert bzw. geschädigt werden, wenn man sie belichtet. Überraschenderweise würde jedoch festgestellt, dass sich funktionelle in vitro-Eigenschaften von TK (z. B. die hypotone Schockreaktion und die beispielsweise durch Collagen induzierte Aggregation) sowie sonstige Thrombozytenparameter nach der Behandlung mit Delta-Ala und anschließender Belichtung nicht verändert hatten.

In den meisten Bakterien entsteht über einen ähnlichen Syntheseweg kein PPIX, sondern CP und eine Reihe anderer Porphyrinverbindungen, die ähnlich aufgebaut sind und ähnliche photochemische Eigenschaften besitzen. Sowohl in tierischen Zellen als auch in Bakterien startet die Synthese mit $\delta$-Aminolävulinsäure (Delta-Ala), das selbst intrazellulär synthetisiert wird, wobei Glycin und Succinyl-Coenzym A die Vorstufen sind. Die enzymatische Transformation erfolgt im Falle von Delta-Ala bzw. deren Derivaten nach Passieren von Membranbarrieren intrazellulär.

Delta-Ala ist somit eine körpereigene Substanz; seine Plasmakonzentration bei Gesunden liegt veröffentlichten Untersuchungen zufolge zwischen 0,024 und 0,27 µM/L.

Es ist seit langem bekannt, dass sich sowohl in aktivierten Zellen als auch in Bakterien die Synthese von PPIX bzw. CP stark steigern lässt, wenn man ihnen Delta-Ala als Substrat anbietet. Hierauf beruhen mehrere Konzepte zur photodynamischen Therapie von Tumoren und von entzündlichen Erkrankungen, z.B. der Haut. Es gibt gleichfalls Konzepte zur photodynamischen Therapie bakterieller Infektionen (siehe z.B. "Photodynamic destruction of Haemophilus parainfluenzae by endogenously produced porphyrins" by van der F.W.Meulen, K.Ibrahim, H.J. Sterenborg, L. V.Alphen, A.Maikoe, J.Dankert in Photochem Photobiol B 1997 Oct;40(3),204-8).

Tumorzellen bzw. Zellen in entzündeten Geweben sind im Allgemeinen stärker aktiviert als nicht entartete Körperzellen und synthetisieren dementsprechend beträchtlich mehr PPIX, wenn man ihnen Delta-Ala zuführt.

Im Allgemeinen befinden sich periphere Blutlymphozyten (PBL) jedoch im Ruhezustand, und so war zu erwarten, dass sich ihnen durch Delta-Ala die Synthese von PPIX in einem Ausmaß, das zur photodynamischen Abtötung ausreicht, nicht induzieren lässt. Dies geht u.a. aus folgenden Veröffentlichungen hervor: D. Grebenova et al., (1998) "Selective destruction of leukaemic cells by photoactivation of 5-aminolevulinic acid-induced protoporphyrin-IX", J. Photochem. Photobiol.B:Biol. 47, 74-78 und E.A. Hryorenko et al. (1998), "Characterization of endogenous protoporphyrin IX induced by delta-aminolevulinic acid in resting and activated peripheral blood lymphocytes by fourcolor flow cytometry", Photochem. Photobiol., 67, 565 - 572.

Im vorliegenden Fall, bei der es um die Behandlung von Thromozytensuspensionen bei etwa Raumtemperatur und nicht bei der für die Proliferation von Zellen optimalen Temperatur von 37 °C geht, erschien dies besonders unwahrscheinlich. Veröffentlichungen zufolge akkumulieren zudem in der Gegenwart von Plasmaproteinen und anderen Plasmabestandteilen die intrazellulär synthetisierten Porphyrine nicht in den Zellen, sondern werden ausgeschleust, wo sie wirkungslos sind, weil ihre Konzentration zu gering ist und weil Plasmabestandteile hemmend wirken (siehe z. B. J. Hanania, Z. Malik "The effect of EDTA and serum on endogenous porphyrin accumulation and photodynamic sensitization of human K562 leukemic cells" Cancer Lett. 65(1992),127-131).

So erschien es auch unwahrscheinlich, dass Bakterien in Thrombozytenkonzentraten inaktivierbar sind, weil, wie bereits erwähnt derzeit eine Mindestkonzentration von etwa 30 % Plasma erforderlich ist, damit sie während der Lagerung ihre Funktionsfähigkeit behalten. Es war deshalb sehr überraschend, dass sowohl periphere Blutlymphozyten als auch Bakterien unter den beschriebenen Bedingungen durchaus nach einer Vorbehandlung mit Delta-Ala und anschließender Belichtung inaktivierbar sind bzw. abgetötet werden können.

Die Behandlung von Thrombozyten-Suspensionen gemäß der Erfindung wird vorzugsweise wie folgt durchgeführt:

Blutspenden werden in der Regel in Form von Verpackungseinheiten mit 450 bis 500 ml Inhalt in speziellen Kunststoffbeuteln aufbewahrt. Auch die betreffenden Thrombozyten-Suspensionen befinden sich in Mengen von ca. 100 bis 1000 ml, vorzugsweise ca. 200 bis 600 ml, in einem transparenten Beutel aus Kunststofffolie, z.B. aus PVC oder Polyolefinen, wie sie üblicherweise zur Herstellung und Lagerung der genannten Blutprodukte verwendet werden.

Der Thrombozyten-Suspension wird Delta-Ala in der benötigten Konzentration zugefügt; dann wird es für eine vorgegebene Zeit bei einer Temperatur inkubiert, die das Eindringen von Delta-Ala in Leukozyten und Bakterien sowie eine ausreichende Porphyrinsynthese ermöglicht.

Bei den durchgeführten Versuchen wurde im Allgemeinen bei Raumtemperatur inkubiert, doch sind auch höhere oder niedrigere Temperaturen möglich. Wichtig ist, dass Delta-Ala in die Zielzellen eindringen kann, dass PPIX und andere Porphyrine synthetisiert und dass FP bzw. Thrombozyten oder Erythrozyten bei der betreffenden Temperatur nicht geschädigt werden.

Anstatt Delta-Ala lassen sich auch Derivate von Delta-Ala einsetzen, z.B. dessen Ester oder die Amide, die wahrscheinlich aufgrund ihrer lipophileren Natur leichter in zelluläre Membranen eindringen und von Zellen aufgenommen werden können als Delta-Ala selbst, insbesondere solche, deren Alkohol- bzw. Amid-Gruppe 1 bis 4 Kohlenstoffatome aufweist. Es sollte bei der Verwendung lipophilerer Abkömmlinge von Delta-Ala aber darauf geachtet werde, dass sich diese nicht in z.B. Thrombozyten all zu sehr anreichern. Geeignete Delta-Ala - Derivate und deren Synthese sind in der Literaturstelle J. Kloek; G.M.J Beijersbergen van Henegouwen; "Prodrugs of 5-aminolevlinic acid for photodynamic therapy"; Photochemistry and Photobiology 64(6),1964,994-1000 beschrieben, die hiermit auch zum Inhalt dieser Anmeldung gemacht wird. Vorsorglich sei erwähnt, dass wenn immer in der vorliegenden Anmeldung auf Delta-Ala Bezug genommen wird, statt Delta-Ala auch ein Delta-Ala-Derivat eingesetzt werden kann bzw. gemeint ist.

Die Einsatzkonzentration von Delta-Ala hängt vor allem von der Plasmakonzentration des zu behandelnden Blutprodukts ab. Beispielsweise liegt bei TK, die oft in nahezu 100 % Plasma als Supendiermedium suspendiert sind, die wirksame Konzentration von Delta-Ala zwischen etwa 0,5 und 5 mM (M=mol/l). Sind die TK in einem Lagermedium mit nur ca. 30 % Plasma suspendiert, reichen ca. 100 bis 1000 µM Delta-Ala aus.

Die erwähnte Vorinkubation zur Akkumulation von PPIX und anderen Porphyrinen kann je nach Temperatur und dem erwünschten Effekt zwischen einigen Minuten und ca. 20 Stunden dauern. Will man beispielsweise nur Bakterien inaktivieren, die eine hohe Teilungsaktivität haben, reichen bei Raumtemperatur ca. 15 min aus.

Will man auch weiße Blutzellen erfassen, die sich im Allgemeinen in der Ruhephase des Zellzyklus befinden, wenig Stoffe aufnehmen und einen geringen Stoffwechsel haben, muss die Vorinkubationsdauer auf mehrere Stunden, z.B. über 16 Stunden, ausgedehnt werden. Dies ist bei TK kein Problem, da diese ohnehin bei Raumtemperatur gelagert werden.

Im Anschluss an die Vorinkubation werden die Thrombozyten-Suspensionen belichtet. Was die Art der verwendeten Lichtquellen betrifft, gibt es mehrere Möglichkeiten, da PPIX, CP und die anderen Porphyrin-Verbindungen, die intrazellulär gebildet werden, mehrere Lichtabsorptionsmaxima im sichtbaren und im ultravioletten Bereich des Spektrums aufweisen. Ein Absorptionsmaximum von PPIX liegt bei 635 nm, also im roten Bereich, weitere Maxima im sichtbaren Bereich liegen zwischen ca. 400 und 580 nm; im langwelligen UV-Bereich (UV-A) gibt es das größte Maximum zwischen ca. 320 und 400 nm. Ähnlich sieht es bei CP aus, das wie erwähnt von Bakterien synthetisiert wird. Vorzugsweise wird Strahlung eingesetzt, welche Wellenlängen im Bereich von 320 bis 580 nm, besonders bevorzugt von 320 bis 500 nm aufweist. Strahlung, die ausschließlich Licht von Wellenlängen über 580 nm aufweist, hat sich für Thrombozyten-Suspensionen überraschend als weniger wirksam erwiesen.

Das Lichtabsorptionsmaximum von CP im roten Bereich des Spektrums liegt allerdings nicht bei 635, sondern bei 617 nm. So wurde denn auch festgestellt, dass Bakterien bei Belichtung mit dieser Wellenlänge effektiver inaktiviert werden als bei 635 nm (F.W. van der Meulen et al. (1997): "Photodynamic destruction of Haemophilus parainfluenzae by endogenously produced porphyrins", J. photochem. Photobiol. B: Biol. 40, 204 - 208). Bei den eigenen Untersuchungen zur Inaktivierung von Bakterien in TK war hingegen keine der beiden Wellenlängen bei im wesentlichen ausschließlichen Einsatz ausreichend genug wirksam. Überraschenderweise zeigte sich aber, dass sich Bakterien und auch Leukozyten in TK nach der Vorbehandlung mit Delta-Ala gut inaktivieren lassen, wenn sie mit weißem Licht bestrahlt werden, wie es beispielsweise Xenonlampen emittieren. Dies wird in den nachfolgenden Versuchsbeispielen demonstriert.

Einsetzbare Lichtquellen können rotes, grünes, blaues, weißes, UV-A oder UV-A- und weißes Licht emittieren, wie es beispielsweise bei Xenonlampen der Fall ist. Will man den UV-A-Anteil des Absorptionsspektrums ausnutzen, so ist darauf zu achten, dass das Folienmaterial, aus dem die verwendeten Kunststoffbehältnisse bestehen, UV-durchlässig ist.

Die Belichtungsdauer hängt vom Produkt ab, das behandelt wird, d.h. von dessen Lichtdurchlässigkeit und der des Folienmaterials des Kunststoffbeutels, in dem es sich befindet, von der eingesetzten Lichtquelle (je intensiver diese ist, umso kürzer muss belichtet werden), von der Konzentration an Delta-Ala und von der Vorinkubationsdauer und -temperatur in Gegenwart von Delta-Ala vor der Belichtung, d.h. von der Menge an PPIX und anderen Porphyrinen, die in den Zielzellen bzw. Bakterien gebildet und angereichert worden sind.

Aus den genannten Gründen ist eine generelle Aussage über die erforderliche Belichtungszeit unabhängig vom Einsatzgut nicht ohne weiteres möglich, und es ist so lange zu belichten, bis die Zielzellen und Mikroorganismen im erforderlichen Ausmaß abgetötet sind.

## Materialien und Methoden

Bei den beschriebenen Versuchen wurden TK verwendet, die aus den Buffy Coats von Spenderblut gewonnen worden waren und die in Plasma bzw. in einem üblichen Lagerungsmedium (T-Sol) mit ca. 40 % Plasma suspendiert waren. Zur Belichtung wurde eine Anlage benutzt, die mit Xenonlampen ausgestattet war und bei der der emittierte UV-Anteil durch einen Filter aus Fensterglas ausgeblendet wurde. Als Testbakterium wurde im Allgemeinen Staphylococcus (S.) epidermidis eingesetzt. Bakterientiter wurden mittels eines Koloniebildungsassays bestimmt. Sie werden als "Koloniebildende Einheiten pro ml" (KBE/ml) angegeben.

Mononukleäre Zellen (MNC) wurden mittels Dichtegradientenzentrifugation über Ficoll® / Hypaque® aus Spenderblut isoliert. Sie wurden bei den Untersuchungen zur Inaktivierung von Leukozyten den TK in einer Konzentration von $7 \times 10^5$/ml zugesetzt. Nach der Behandlung wurden Aliquots der Zellsuspensionen niedrigtourig zentrifugiert (1500 rpm bzw. 600 g für 4 min).

Die pelletierten Zellen wurden drei Mal mit Zellkulturmedium gewaschen (RPMI 1640 mit 10 % fötalem Kälberserum und Antibiotika) und dann bei einer Zellkonzentration von wiederum $7 \times 10^5$/ml im selben Medium resuspendiert. Die Vitalität der Zellen wurde mittels eines Proliferationsassays geprüft. Hierbei wurden die Zellen mit Concanavalin A (Con A, 2 µg/ml) stimuliert und in Aliquots von 200 µl für 3 bis 4 Tage bei 37 °C im $CO_2$-begasten Brutschrank kultiviert. Dann wurde den Zellsuspensionen Brom-Deoxyuridin (BRDU) zugegeben. Anschließend wurden sie für 4 Stunden weiterkultiviert, und danach wurde die Einbaurate von BRDU spektralphotometrisch bei einer Wellenlänge von 450 nm bestimmt. Die Extinktionswerte ($OD_{450}$) bei dieser Wellenlänge sind dem Einbau von BRDU in die zelluläre DNS und damit der Viabilität der Zellen proportional.

## Versuchsbeispiele

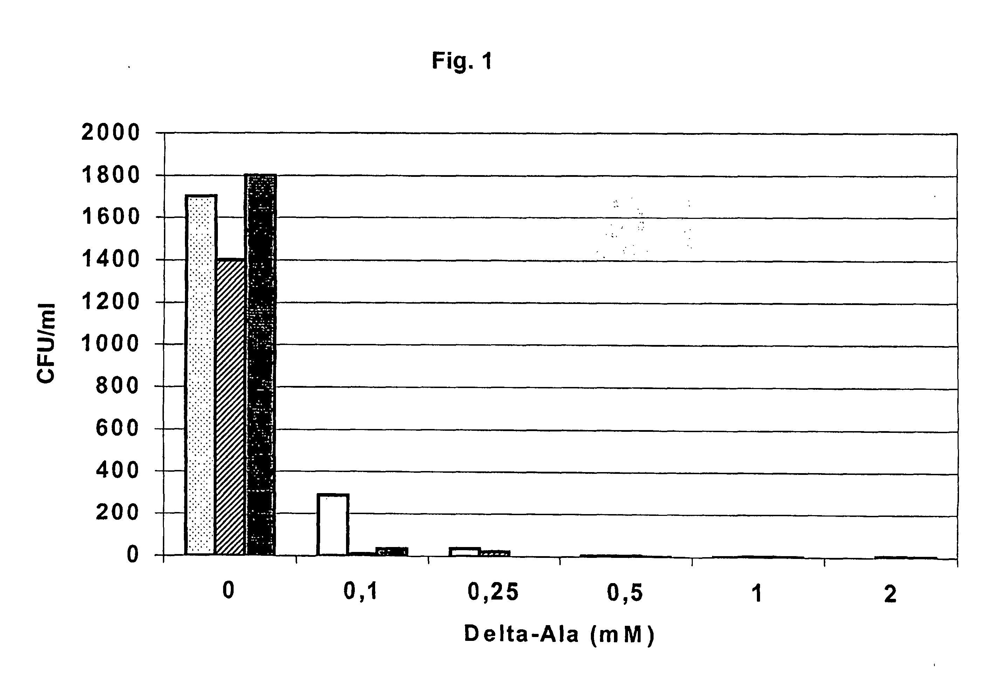

Bei den Versuchen zur Inaktivierung von Bakterien wurden Aliquots von TK von jeweils 60 ml behandelt, die sich in Lagerbeuteln aus PVC mit einem Nennvolumen von 500 ml befanden. Die Proben wurden nach der Zugabe von Delta-Ala bzw. dem Methylester von Delta-Ala (siehe die Tab. 1) für eine Stunde bei Raumtemperatur im Thrombozytenrotator vorinkubiert und dann belichtet. Bei den Versuchen zur Inaktivierung von Leukozyten wurden die Proben über Nacht (d.h. für ca. 16-20 h) bzw. für 4 Stunden vorinkubiert. Die in die Proben eingebrachte Lichtenergie ist in Kilojoule pro m$^2$ (kJ/m$^2$) angegeben. 3000 kJ/m$^2$ entsprechen einer Belichtungsdauer von etwa einer Stunde. Jeder Versuch wurde mindestens zwei Mal durchgeführt. Die Ergebnisse der Versuche sind in den Tabellen 1 und 2 in den aufgeführt und Fig. 1 bis 5 wiedergegeben. Die Ergebnisse von Versuchswiederholungen sind dort durch unterschiedliche Farben der Balken gekennzeichnet.

**Photoinaktivierung von Bakterien**

Abhängigkeit von der Delta-Ala-Konzentration

In Fig. 1 ist die Abhängigkeit der Photoinaktivierung von S. epidermidis in TK von der Delta-Ala -Konzentration dargestellt. Die eingetragene Lichtenergie war 3000 kJ/m$^2$.

Wie aus dem Ergebnis hervorgeht, wurde S. epidermidis weitgehend inaktiviert, wenn die TK mit Delta-Ala im Konzentrationsbereich zwischen ca. 0,5 und 1 mM vorinkubiert und dann intensiv belichtet wurden. In plasmareduzierten TK reichten unter ansonsten gleichen Versuchsbedingungen bereits Delta-Ala -Konzentrationen von 0,1 mM und weniger aus. Dargestellt sind die Ergebnisse von 3 Versuchen.

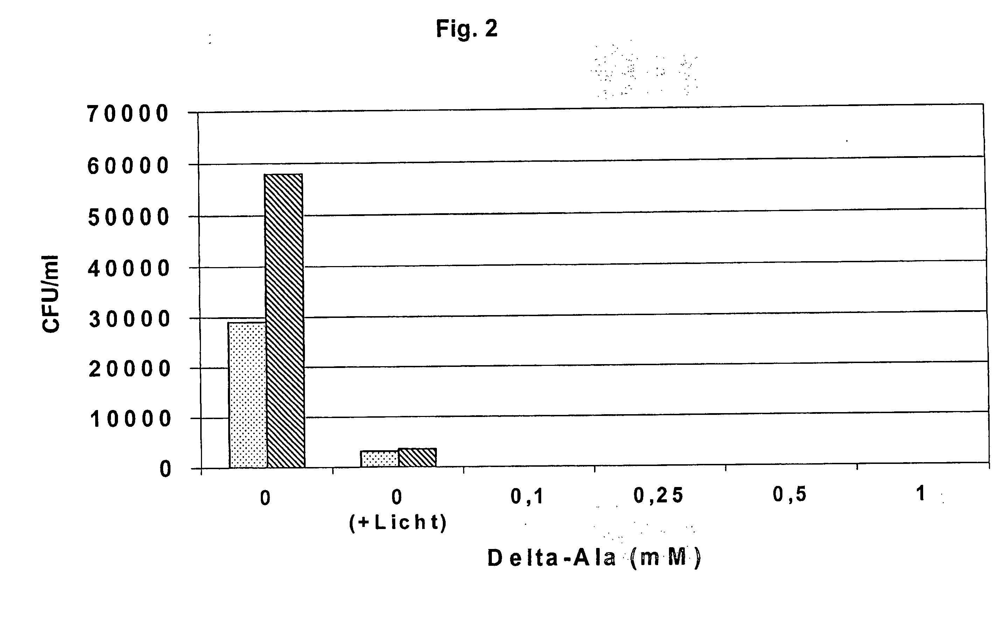

In Fig. 2 ist die Abhängigkeit der Photoinaktivierung von S. epidermidis in plasmareduzierten TK von der Delta-Ala - Konzentration dargestellt. Die eingetragene Lichtenergie war wiederum 3000 kJ/m$^2$. Dargestellt sind die Ergebnisse von 2 Versuchen. Auch die Belichtung allein führte schon zu einer merklichen Reduktion des Bakterientiters.

Kinetik der Photoinaktivierung von S. epidermidis

Bei den Versuchen, deren Ergebnisse in den Fig. 1 und 2 dargestellt sind, wurden die TK mit 3000 kJ/m$^2$ belichtet, was sicherlich mehr war, als eigentlich benötigt wird. Um festzustellen, welche Lichtmenge ausreicht, um S. epidermideis weitgehend zu inaktivieren, d.h. um wenigstens 3 $\log_{10}$-Stufen, wurden die folgenden Experimente durchgeführt: TK, entweder in Lagermedium mit einem Plasmagehalt von ca. 40 % oder in Plasma, wurden 1000 bis 1500 CFU/ml S. epidermidis zugefügt; dann wurde Delta-Ala in einer Konzentration von 0,25 mM (plasmareduzierte TK) bzw. 1 mM (TK mit ca. 100 % Plasma) zugegeben. Nach einer einstündigen Vorinkubation bei Raumtemperatur wurden die Proben mit verschiedenen Lichtmengen bestrahlt. Die Ergebnisse zeigen die Fig. 3 und 4.

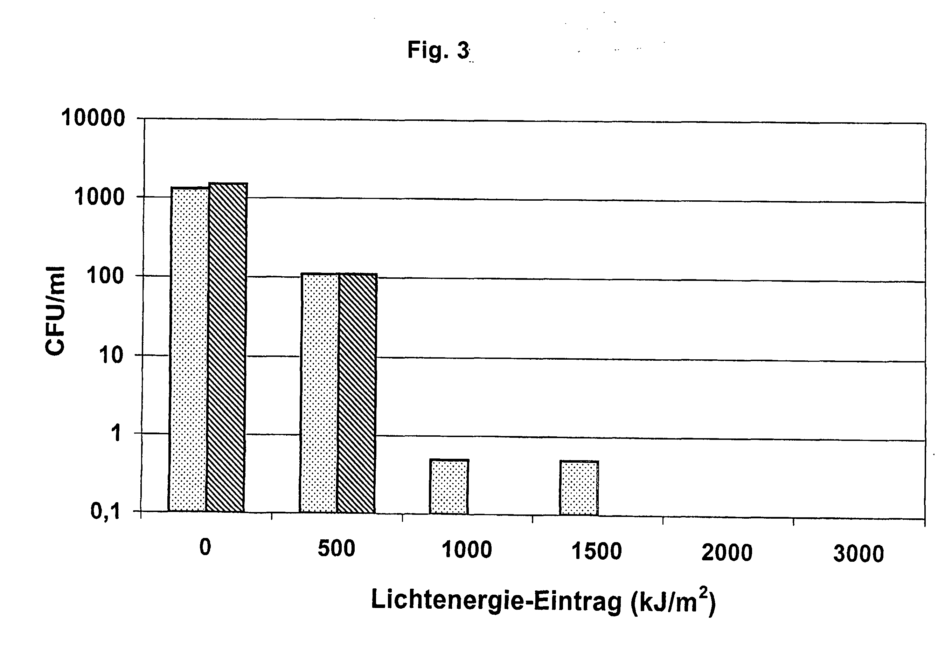

In Fig. 3 ist die Kinetik der Photoinaktivierung von S. epidermidis in plasmareduzierten TK nach einstündiger Präinkubation in der Gegenwart von 0,25 mM Delta-Ala dargestellt.

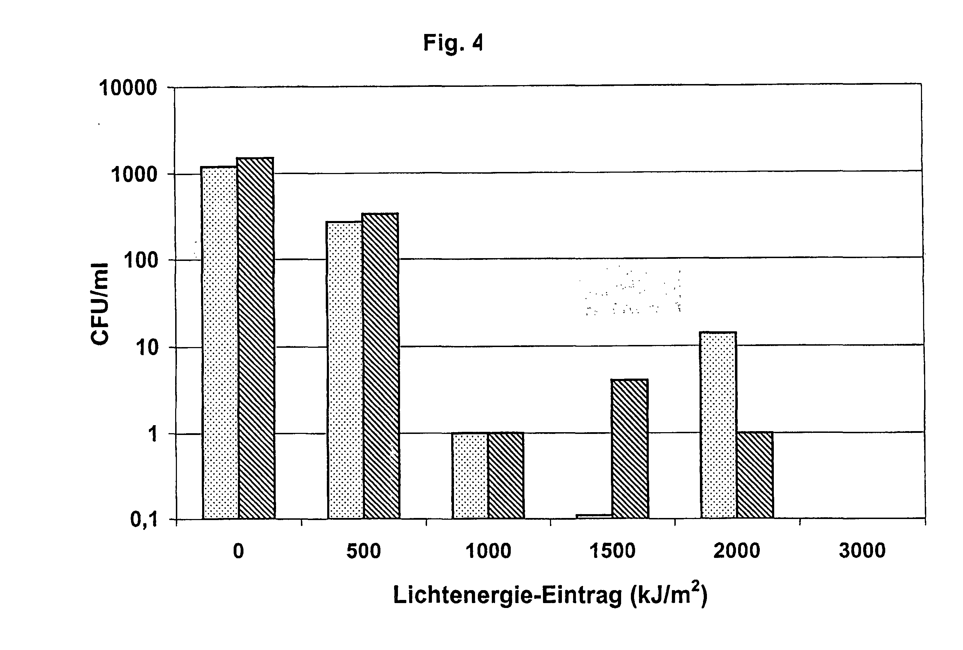

Fig. 4 gibt die Kinetik der Photoinaktivierung von S. epidermidis in TK nach einstündiger Präinkubation in der Gegenwart von 1 mM Delta-Ala wieder.

Aus den Fig. 3 und 4 lässt sich als Ergebnis festhalten, dass für die plasmareduzierten TK etwa 1500 bis 2000 kJ/m$^2$ zur weitgehenden Inaktivierung der Bakterien ausreichten, während für Thrombozyten, die in 100% Plasma suspendiert waren, eine Lichtmenge von ca. 3000 kJ/m$^2$ eingestrahlt werden sollte.

Verwendung des Methylester von Delta-Ala anstelle von Delta-Ala

Wie aus der Tab. 1 hervorgeht, hat der Methylester von Delta-Ala die gleiche Wirksamkeit, was die Inaktivierung von S. epidermidis angeht. Dies belegt, dass Abkömmlinge von Delta-Ala gleichfalls in photoaktive Substanzen umgesetzt werden und dass man sie für den beabsichtigten Zweck, d.h. zur Dekontamination von Blutkomponenten, ebenfalls einsetzen kann.

Tab. 1 :

*Photoinaktivierung von S. epidermidis in* TK in 100% Plasma. *Vergleich von Delta-Ala mit Delta-Ala - Methylester (Methyl-Ala). Beide Verbindungen wurden in einer Konzentration von 2 mM eingesetzt. Lichtenergie-Eintrag: 3000 kJ/m$^2$. Die Kontrollprobe wurde nicht belichtet.*

| **Zugefügte Verbindung** | **Bakterientiter nach Licht (CFU/ml)** | |
|---|---|---|
| | **Exp. 1** | **Exp. 2** |
| Kontrolle | 250 | 210 |
| Delta-Ala | 5 | 3,5 |

Tab. 1 : (fortgesetzt)

| Zugefügte Verbindung | Bakterientiter nach Licht (CFU/ml) | |
|---|---|---|
| | Exp. 1 | Exp. 2 |
| Methyl-Ala | 1,5 | 4 |

Photoinaktivierung weiterer Bakterien

Aus der Tab. 2 geht hervor, dass neben S. epidermidis auch weitere gram-positive und gram-negative Bakterien in TK inaktiviert werden können, wenn sie mit Delta-Ala vorbehandelt und anschließend belichtet werden. E. cloacae und S. aureus sind offenbar etwas resistenter als die anderen Testbakterien; aber auch bei ihnen werden unter den gewählten experimentellen Bedingungen die infektiösen Titer um ca. 95 bzw. 97 % reduziert. Aus den Ergebnissen der vorherigen Untersuchungen lässt sich ableiten, dass alle Bakterien völlig inaktiviert werden können, wenn die Belichtungszeit verlängert oder die Konzentration von Delta-Ala erhöht wird.

Tab: 2

| *Photoinaktivierung verschiedener Bakterien in TK nach Vorbehandlung mit 1 mM Delta-Ala (Mittelwerte aus jeweils zwei Versuchen), Lichtenergie - Eintrag: 1500 kJ/m$^2$.* | | |
|---|---|---|
| **Bakterium** | **Gram- Färbung** | **Bakterien - Titer (CFU/ml)** vor Belichtung / nach Belichtung |
| Staphylococcus epidermidis | + | 2440 | 2 |
| Pseudomonas aeroginosa | - | 700 | 1 |
| Staphylococcus aureus | + | 2000 | 51 |
| Yersinia enterocolitica | - | 4650 | 1 |
| Escherichia coli | - | 11300 | 3 |
| Serratia marescens | - | 2500 | 1 |
| Enterobacter cloacae | - | 24500 | 1280 |

## Photoinaktivierung von weißen Blutzellen

Abhängigkeit von der Delta-Ala Konzentration

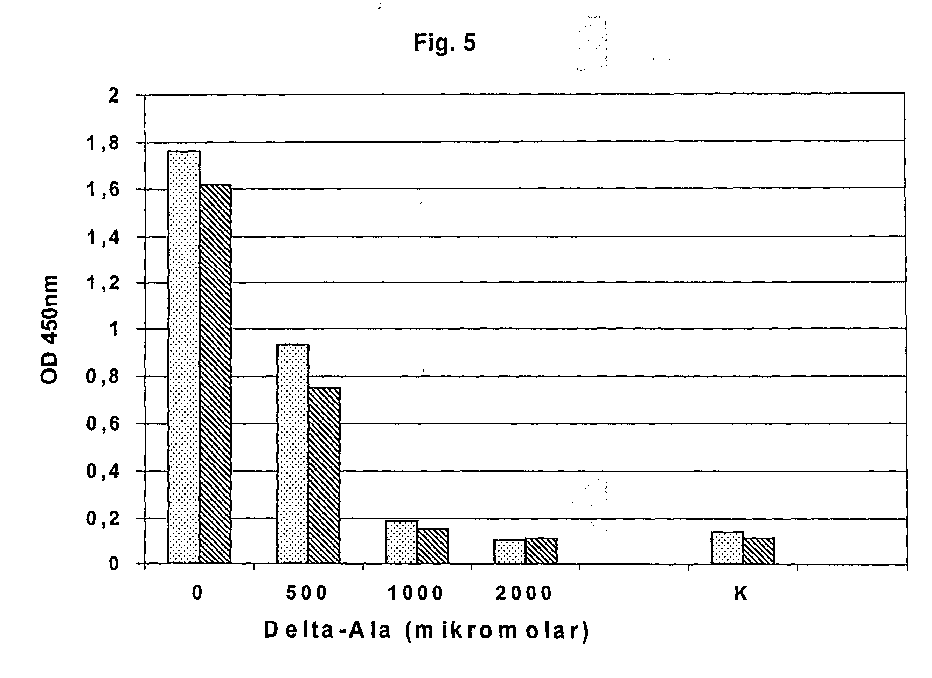

In Fig. 5 ist die Photoinaktivierung von T-Lymphozyten in TK in Abhängigkeit von der Konzentration von Delta-Ala dargestellt (Konzentrationsbereich: 500-2000 µM, Vorinkubationsdauer: ca. 16 h; Lichtenergie-Eintrag: 2000 kJ/m$^2$, K = unstimulierte Kontrollprobe). Die erforderliche Konzentration von Delta-Ala liegt offenbar oberhalb von 1 mM. Im Unterschied zu Bakterien, die nur für kurze Zeit vorinkubiert werden müssen (bei den vorstehenden Untersuchungen betrug die Präinkubationsdauer immer eine Stunde; 15-30 Minuten reichen aber auch aus), ist es in der Regel notwendig, die Präinkubationsdauer auf mehrere Stunden auszudehnen, damit die Zellen durch einen Lichtenergie-Eintrag von 1000-2000 kJ/cm$^2$ weitgehend inaktiviert werden können.

## Patentansprüche

**1.**   Verfahren zur Inaktivierung von Bakterien und/oder Leukozyten in aus Blut oder Blutprodukten erhältlichen Suspensionen enthaltend Thrombozyten durch photodynamische Behandlung umfassend die folgenden Schritte:

(a) Bereitstellen der Suspension enthaltend Thrombozyten,
(b) Zugabe zumindest einer Vorläufersubstanz zur Synthese zumindest einer photoaktiven Substanz, wobei es sich bei der Vorläufersubstanz zumindest um δ-Aminolävulinsäure bzw. um ein Derivat der δ-Aminolävulinsäure handelt,
(c) Induktion zur intrazellulären Synthese einer oder mehrer photoaktiver Substanzen und
(d) Aussetzen der Suspension enthaltend Thrombozyten einer Bestrahlung in zumindest einem Absorptionswellenlängenbereich der photoaktiven Substanz(en),

**dadurch gekennzeichnet, dass**

- die Suspension enthaltend Thrombozyten eine durch Aufreinigen und Aufkonzentrieren von Blut oder Blutprodukten erhältliches Thrombozytenkonzentrat ist, welches zumindest $5 \times 10^8$ Thrombozyten pro ml enthält und

das Verfahren durch zumindest eines der folgenden Merkmale weiterhin **gekennzeichnet** ist:

- Inkubieren des Thrombozytenkonzentrates bei 15 bis 28°C zusammen mit der Vorläufersubstanz zur Bildung der photoaktiven Substanz(en) und/oder
- die Bestrahlung in zumindest einem Absorptionswellenlängenbereich der photoaktiven Substanz(en) (Schritt (d)) eine Bestrahlung mit zumindest Licht im Wellenlängenbereich von 320 nm bis 580 nm umfasst, ggf. auch monochromatisches Licht dieses Wellenlängenbereichs.

2. Verfahren gemäß Anspruch 1, wobei es sich bei der Vorläufersubstanz um Ester oder Amide der δ-Aminolävulinsäure handelt.

3. Verfahren gemäß einem der vorhergehenden Ansprüche, **dadurch gekenn- zeichnet, dass** die Vorläufersubstanz in einer Menge zugesetzt wird, so dass sich eine Konzentration von 0,01 bis 10 mmol pro Liter, vorzugsweise zwischen 0,1 und 5 mmol pro Liter, ausbildet.

4. Verfahren gemäß einem der vorhergehenden Ansprüche, **dadurch gekenn-** zeichnet, dass die Vorläufersubstanz zumindest 15 Minuten, vorzugsweise 1 Stunde, vor der Bestrahlung zugesetzt wird.

5. Verfahren gemäß einem der vorhergehenden Ansprüche, **dadurch gekenn- zeichnet, dass** die Bestrahlung mit Licht im Bereich von Wellenlängen zumindest eines Absorptionsmaximums, vorzugsweise mehrerer Absorptionsmaxima der photoaktiven Substanzen durchgeführt wird, deren Synthese induziert wurde.

6. Verfahren gemäß einem der vorhergehenden Ansprüche, **dadurch gekenn- zeichnet, dass** das Thrombozytenkonzentrat zusammen mit der Vorläufersubstanz bei 18 bis 25°C zur Bildung der photoaktiven Substanz(en) inkubiert wird.

7. Verfahren gemäß einem der vorhergehenden Ansprüche, **dadurch gekenn-** zeichnet, dass die Bestrahlung mit Lichtenergie von 100 bis 10.000 kJ/m$^2$, vorzugsweise von 100 bis 3000 kJ/m$^2$, besonders bevorzugt von 500 bis 2000 kJ/m$^2$ erfolgt.

8. Verfahren gemäß einem der vorhergehenden Ansprüche, **dadurch gekenn- zeichnet, dass** das Thrombozytenkonzentrat Plasma und ggf. ein geeignetes Lagerungsmedium enthalten, wobei der Plasmagehalt vorzugsweise größer 20 Gew.% beträgt.

9. Verfahren gemäß einem der vorhergehenden Ansprüche, **dadurch gekenn-** zeichnet, dass die Behandlung des Thrombozytenkonzentrates in Blutbeuteln erfolgt.

10. Verfahren gemäß einem Ansprüche 1 bis 8, **dadurch gekennzeichnet, dass** die Behandlung des Thrombozytenkonzentrates in einer Durchflussapparatur erfolgt.

11. Verfahren gemäß einem der vorhergehenden Ansprüche, **dadurch gekenn- zeichnet, dass** die Leukozyten T-Lymphozyten sind.

## Claims

1. A process for inactivating bacteria and/or leukocytes in suspensions comprising thrombocytes obtainable from blood or blood products by photodynamic treatment comprising the following steps:

    (a) Providing the thrombocyte suspension,
    (b) Addition of at least one precursor substance for the synthesis of at least one photoactive substance, wherein the precursor substance is at least δ-aminolevulinic acid or a derivative of δ-aminolevulinic acid,

(c) Induction for the intracellular synthesis of one or a plurality of photoactive substances and
(d) Exposure of the suspension comprising thrombocytes to irradiation in at least one absorption wavelength range of the photoactive substance(s);

**characterized in that**

- the suspension comprising thrombocytes is a thrombocyte concentrate obtainable from blood or blood products by purification and concentration comprising at least $5 \times 10^8$ thrombocytes per ml and

the process is further **characterized by** at least one of the following features

- incubation of the thrombocyte concentrate at 15 to 28 °C together with the precursor substance to form the photoactive substance(s) and/or
- the irradiation in at least one absorption wavelength range of the photoactive substance(s) (step (d)) comprises an irradiation with at least light in the wavelength range from 320 nm to 580 nm, optionally monochromatic light of said wavelength range.

2. The process according to claim 1, wherein the precursor substance is an ester or amide of δ-aminolevulinic acid.

3. The process according to any one of the preceding claims, **characterised in that** the precursor substance is added in a quantity such that a concentration of 0.01 to 10 mmol per litre, preferably between 0.1 and 5 mmol per litre, is formed.

4. The process according to any one of the preceding claims, **characterised in that** the precursor substance is added at least 15 minutes, preferably 1 hour, prior to the irradiation.

5. The process according to any one of the preceding claims, **characterised in that** the irradiation is carried out using light in the range of wavelengths of at least one absorption maximum, preferably a plurality of absorption maxima, of the photoactive substances whose synthesis was induced.

6. The process according to any one of the preceding claims, **characterised in that** the thrombocyte concentrate together with the precursor substance is incubated at 18 to 25 °C to form the photoactive substance(s).

7. The process according to any one of the preceding claims, **characterised in that** the irradiation is carried out using light energy of 100 to 10000 kJ/cm$^2$, preferably of 100 to 3000 kJ/cm$^2$, in particular preferred of 500 to 2000 kJ/m$^2$.

8. The process according to any one of the preceding claims, **characterised in that** the thrombocyte concentrate contains plasma and optionally a suitable storage medium, wherein the plasma content is preferably higher than 20 wt. %.

9. The process according to any one of the preceding claims, **characterised in that** the treatment of the concentrate takes place in blood bags.

10. The process according to any one of claims 1 to 8, **characterised in that** the treatment of the thrombocyte concentrate takes place in a continuous flow apparatus.

11. The process according to any one of the preceding claims, **characterised in that** the leukocytes are T lymphocytes.

**Revendications**

1. Procédé d'inactivation de bactéries et/ou de leucocytes dans des suspensions obtenues à partir de sang ou de produits de sang qui contiennent des thrombocytes, par un traitement photodynamique qui comprend les étapes ci-dessous:

(a) préparation de la suspension qui contient des thrombocytes,
(b) addition d'au moins une substance qui est un précurseur d'au moins une substance photoactive, la substance de précurseur étant au moins l'acide δ-aminolévulinique ou selon le cas un dérivé de l'acide δ-aminolévulinique,
(c) induction d'une ou de plusieurs substances photoactives en vue de la synthèse intracellulaire et
(d) exposition de la suspension qui contient des thrombocytes à une irradiation dans au moins une plage de longueurs d'onde absorbées par la ou les substances photoactives,

**caractérisé en ce que**

- la suspension qui contient des thrombocytes est un concentré de thrombocytes obtenu par purification et concentration de sang ou de produits de sang et qui contient au moins $5 \times 10^8$ thrombocytes par ml,

le procédé étant en outre **caractérisé par** au moins l'une des caractéristiques ci-dessous:

- l'incubation du concentré de thrombocytes entre 15 et 28°C est réalisée en même temps que la substance qui forme un précurseur de formation de la ou des substances photoactives et/ou
- l'irradiation dans au moins une plage de longueurs d'onde absorbées par la ou les substances photoactives (étape (d)) comporte une irradiation par au moins de la lumière dans la plage de longueurs d'onde de 320 nm à 580 nm, et éventuellement aussi par une lumière monochromatique dans cette plage de longueur d'onde.

2. Procédé selon la revendication 1, dans lequel la substance de précurseur est constituée d'un ester ou d'un amide de l'acide δ-aminolévulinique.

3. Procédé selon l'une quelconque des revendications précédentes, **caractérisé en ce que** la substance de précurseur est ajoutée en une quantité telle qu'on en forme une concentration de 0,01 à 10 mmoles par litre, de préférence de 0,1 à 5 mmoles par litre.

4. Procédé selon l'une quelconque des revendications précédentes, **caractérisé en ce que** la substance de précurseur est ajoutée au moins 15 minutes et de préférence 1 heure avant l'irradiation.

5. Procédé selon l'une quelconque des revendications précédentes, **caractérisé en ce que** l'irradiation est réalisée avec de la lumière dans la plage des longueurs d'onde qui contient au moins un maximum d'absorption et de préférence plusieurs maxima d'absorption par les substances photoactives, dont la synthèse a été induite.

6. Procédé selon l'une quelconque des revendications précédentes, **caractérisé en ce que** pour former la ou les substances photoactives, le concentré de thrombocytes est incubé entre 18 et 25°C en même temps que la substance de précurseur.

7. Procédé selon l'une quelconque des revendications précédentes, **caractérisé en ce que** l'irradiation est réalisée à une énergie lumineuse de 100 à 10 000 kJ/m$^2$, de préférence de 100 à 3 000 kJ/m$^2$ et de manière particulièrement préférée de 500 à 2 000 kJ/m$^2$.

8. Procédé selon l'une quelconque des revendications précédentes, **caractérisé en ce que** le concentré de thrombocytes contient du plasma et éventuellement un milieu de conservation approprié, la teneur en plasma étant de préférence supérieure à 20% en poids.

9. Procédé selon l'une quelconque des revendications précédentes, **caractérisé en ce que** le traitement du concentré de thrombocytes s'effectue dans des pochettes de sang.

10. Procédé selon l'une quelconque des revendications 1 à 8, **caractérisé en ce que** le traitement du concentré de thrombocytes s'effectue dans un appareil traversé par un écoulement du concentré.

11. Procédé selon l'une quelconque des revendications précédentes, **caractérisé en ce que** les leucocytes sont des lymphocytes T.

Fig. 1

**Fig. 2**

EP 1 458 417 B1

Fig. 3

Lichtenergie-Eintrag (kJ/m$^2$)

CFU/ml

Fig. 4

EP 1 458 417 B1

## Fig. 5